# EUROPÄISCHE PATENTSCHRIFT

(11) **EP 4 182 426 B1**
(45) Veröffentlichungstag und Bekanntmachung des Hinweises auf die Patenterteilung: **29.01.2025**
(21) Anmeldenummer: 21746669.7
(22) Anmeldetag: 13.07.2021
(51) Int. Cl.: C12M 1/00, C12N 1/06, C12N 15/10

(54) **LYSE EINER PROBE MITTELS MAGNETELEMENTEN UND ROTATORISCHER RELATIVBEWEGUNG**
LYSIS OF A SAMPLE BY MEANS OF MAGNET ELEMENTS AND ROTATIONAL RELATIVE MOVEMENT
LYSE D'UN ÉCHANTILLON AU MOYEN D'ÉLÉMENTS MAGNÉTIQUES ET D'UN MOUVEMENT DE ROTATION RELATIF

(30) Priorität: 17.07.2020 DE 102020209001
(43) Veröffentlichungstag der Anmeldung: 24.05.2023
(73) Patentinhaber: Hahn-Schickard-Gesellschaft für angewandte Forschung e. V., 70569 Stuttgart (DE)
(72) Erfinder: SCHLANDERER, Judith, 79098 Freiburg (DE); PAUST, Nils, 79115 Freiburg im Breisgau (DE); SCHWEMMER, Frank, 79115 Freiburg (DE); CZILWIK, Gregor, 79104 Freiburg (DE)
(74) Vertreter: Stöckeler, Ferdinand
(86) Internationale Anmeldenummer: PCT/EP2021/069461
(87) Internationale Veröffentlichungsnummer: WO 2022/013220

(56) Entgegenhaltungen:
- EP-A1- 3 222 710
- CN-A- 110 938 522
- US-A1- 2014 087 359
- US-B2- 10 138 458
- GRUMANN M ET AL: "Batch-mode mixing on centrifugal microfluid platforms", LAB ON A CHIP, ROYAL SOCIETY OF CHEMISTRY, UK, vol. 5, 1 January 2005 (2005-01-01), pages 560 - 565, XP002508481, ISSN: 1473-0197, DOI: 10.1039/B418253G

## Beschreibung

### Gebiet

Die vorliegende Offenbarung befasst sich mit Vorrichtungen und Verfahren zur Lyse einer Probe mittels Magnetelementen und rotatorischer Relativbewegung und insbesondere auf entsprechende Vorrichtungen und Verfahren auf dem Gebiet der zentrifugalen Mikrofluidik.

In der Biologie und der Medizin werden zu Forschungszwecken Mikroorganismen durch mechanische Reibe-, Stoß- und Scherkräfte aufgeschlossen, um zum Inneren der Mikroorganismen zu gelangen. Beispielsweise können Zellen aktiv aufgebrochen werden, um an Proteine und/oder DNA im Zellinneren zu gelangen. Ein solches Aufschließen von Mikroorganismen wird auch als Lyse bezeichnet. Vorrichtungen und Verfahren, um ein solches Aufschließen von Mikroorganismen zu bewirken, können somit als Lysevorrichtungen und Lyseverfahren bezeichnet werden.

### Stand der Technik

Es sind unterschiedliche Verfahren zum Aufschießen von Mikroorganismen durch mechanische Reibe-, Stoß- und Scherkräfte bekannt.

Verfahren mit translatorischer, radialer Bewegung magnetischer Aktuatoren in zentrifugal mikrofluidischen Systemen sind Kido et al. [1] und der CA 2827614 C zu entnehmen. Kido et al. [1] beschreiben ein zentrifugal mikrofluidisches Verfahren zur Zelllyse, in welchem Scher- und Reibungskräfte durch eine translatorische, radiale Bewegung des magnetischen Aktuators realisiert werden. In der CA 2827614 C wird ein scheibenförmiger Aktuator ebenfalls in translatorischer, radialer Bewegung durch externe Magnetkräfte in einer Kammer bewegt. Die Funktionalität beider Ansätze ist ähnlich.

Ein Verfahren mit translatorischer, azimutaler Bewegung magnetischer Aktuatoren in einem zentrifugal mikrofluidischen System wird von Siegrist et. al [2] beschrieben. Im Gegensatz zu den bisher beschriebenen Systemen offenbaren Siegrist et. al [2] ein zentrifugal mikrofluidisches Verfahren, bei dem die Reibung der Glaspartikel durch eine translatorische, azimutale Bewegung realisiert wird.

Ein Verfahren mit translatorischer Bewegung magnetischer Aktuatoren in einem nicht zentrifugalen mikrofluidischen System wird im Patent US 8356763 B2 offenbart. Im Patent US 8356763 B2, als nicht zentrifugales mikrofluidisches Lysesystem, ist ein System beschrieben, das die magnetische Aktuierung durch An-, Aus- und Umschalten von Elektromagneten erzeugt. Dabei wird der magnetische Aktuator translatorisch bewegt, eine Rotationsbewegung wird durch die Kammer verhindert.

Ein Verfahren mit translatorischer und rotatorischer Bewegung von magnetischem Aktuator bei einem nicht zentrifugal mikrofluidischen System ist in der US 10138458 B2 offenbart, in der ein Verfahren zur Lyse von Zellen beschrieben ist. Drehende externe Magnete erzeugen ein variierendes Magnetfeld, durch das der magnetische Aktuator mit Rotationsbewegungen, Translationsbewegungen oder einer Kombination dieser Bewegungen beaufschlagt wird.

Ferner ist aus CN 110 938 522 A eine tragbare Lysevorrichtung bekannt, die auf einer zentrifugalen mikrofluidischen Technologie basiert, und ein Verfahren zu ihrer Verwendung. Grumann et al. offenbaren ferner in "Batch-mode mixing on centrifugal microfluid patforms", Lab on a Chip 5.5 (2005): 560-565, einen Aufbau in Form einer mikrostrukturierten Scheibe mit einer runden Mischkammer, die auf einer makroskopischen Antriebseinheit rotiert.

### Überblick

Die Erfinder haben erkannt, dass die aus dem Stand der Technik bekannten Verfahren zur mechanischen Lyse von Mikroorganismen an einigen Nachteilen leiden. So nutzt keiner der zentrifugal mikrofluidischen Ansätze alle möglichen Freiheitsgrade zur Bewegung des magnetischen Aktuators. Dadurch wird nicht das volle Potential von möglichen Kollisionen zwischen magnetischem Aktuator, Partikeln und den Mikroorganismen ausgeschöpft. Im nicht zentrifugalen System, wie es in der US 10138458 B2 beschrieben ist, werden die Freiheitsgrade zum Teil ausgeschöpft. Hierfür wird jedoch ein komplizierter Aufbau benötigt, der ausschließlich für den Schritt der Lyse von Mikroorganismen eingesetzt werden kann. Die Handhabung aller weiteren Schritte muss manuell erfolgen.

Die der vorliegenden Offenbarung zugrundeliegende Aufgabe besteht darin, einen verbesserten Kompromiss zwischen der Effizienz der Lyse und dem Handhabungsaufwand zu erzielen.

Diese Aufgabe wird durch eine Vorrichtung nach Anspruch 1 und ein Verfahren nach Anspruch 15 gelöst.

Beispiele der vorliegenden Offenbarung schaffen eine Lysevorrichtung, die eine Kammer zur Aufnahme einer Probe und zumindest einen magnetischen Aktuator, der sich innerhalb der Kammer befindet, aufweist, sowie zumindest zwei außerhalb der Kammer angeordneten Magnetelemente, die bspw. als Permanent- oder Elektromagnete ausgeführt sein können. Die Kammer ist eine Lysekammer.

Der magnetische Aktuator innerhalb der Kammer kann bspw. als Permanentmagnet ausgeführt sein. Darüber hinaus weist eine solche Lysevorrichtung eine Antriebseinrichtung auf, zum Bewirken einer rotatorischen Relativbewegung zwischen der Kammer und den zumindest zwei außerhalb der Kammer angeordneten Magnetelementen, wobei die Polung der Magnetelemente in Bezug auf die Kreisbahn der rotatorischen Relativbewegung, und somit der Kammer, entgegengesetzt ist, so dass der in der Kammer angeordnete magnetische Aktuator sowohl translatorisch als auch rotatorisch bewegt wird, um eine Lyse der Probe zu bewirken. Dabei ist die Kammer, bspw. durch ihre Dimensionierung oder eine flexible Außenhülle, ausgelegt, um zu ermöglichen, dass sich der zumindest eine magnetische Aktuator, der sich innerhalb der Kammer befindet, sowohl translatorisch, als auch rotatorisch, um eine eigene Achse des magnetischen Aktuators bewegt.

Beispiele der vorliegenden Offenbarung schaffen ein Lyseverfahren, bei dem eine Probe in eine Lysekammer, eingebracht wird, wobei sich in der Kammer zumindest ein magnetischer Aktuator, befindet, der bspw. als Permanentmagnet ausgeführt ist, und wobei die Kammer ausgelegt ist, bspw. durch ihre Dimensionierung oder eine flexible Außenhülle, um zu ermöglichen, dass sich der zumindest eine magnetische Aktuator, der sich innerhalb der Kammer befindet, sowohl translatorisch als auch rotatorisch bewegt.

Bei dem Verfahren wird durch eine Antriebseinrichtung eine rotatorische Relativbewegung zwischen der Kammer, in der sich die Probe und zumindest ein magnetischer Aktuator befindet, und zumindest zwei außerhalb der Kammer angeordneten Magnetelementen bewirkt, wobei die Polung der zumindest zwei Magnetelemente außerhalb der Kammer in Bezug auf die Kreisbahn der rotatorischen Relativbewegung und somit der Kammer entgegengesetzt ist, so dass der in der Kammer angeordnete magnetische Aktuator sowohl translatorisch als auch rotatorisch, um eine eigene Achse des magnetischen Aktuators bewegt wird, um eine Lyse der Probe zu bewirken. Die zumindest zwei außerhalb der Kammer angeordneten Magnetelemente können dabei bspw. als Permanent- oder Elektromagnete ausgeführt sein.

Beispiele der vorliegenden Offenbarung beruhen auf dem Kerngedanken bei einer zentrifugalen, mechanischen Lysevorrichtung mit Hilfe einer rotatorischen Relativbewegung zwischen zumindest zwei Magnetelementen außerhalb einer Kammer und der Kammer, welche eine zu lysierende Probe und zumindest einen magnetischen Aktuator enthält, den magnetischen Aktuator innerhalb der Kammer sowohl translatorisch als auch rotatorisch zu bewegen. Es wurde erkannt, dass die Translation und Rotation des magnetischen Aktuators innerhalb der Kammer durch die Polung der zumindest zwei Magnetelemente außerhalb der Kammer ermöglicht wird, indem die zumindest zwei Magnetelemente außerhalb der Kammer in Bezug auf die Kreisbahn der rotatorischen Relativbewegung, und somit die Kammer, entgegengesetzt gepolt sind. Dadurch kann bewirkt werden, dass der magnetische Aktuator innerhalb der Kammer nicht nur eine Translation, sondern auch eine Rotation ausführt. Bei Beispielen wird die rotatorische Relativbewegung bewirkt, indem die Kammer um eine Rotationsachse rotiert. Bei Beispielen wird die rotatorische Relativbewegung bewirkt, indem die zwei Magneten außerhalb der Kammer um dieselbe Rotationsachse rotieren.

Bei Beispielen können durch die Ausnutzung aller Bewegungsfreiheitsgrade des magnetischen Aktuators innerhalb der Kammer beispielsweise auch schwer lysierbare Proben mit geringem Zeitaufwand lysiert werden. Durch die effizientere Lyse kann beispielsweise auch eine Verkleinerung des Bauraums einer solchen Lysevorrichtung realisiert werden. Durch die Ausnutzung aller Bewegungsfreiheitsgrade in einer zentrifugalen mechanischen Vorrichtung kann diese effiziente Form der Lyse beispielsweise auch einfacher in eine Vorrichtung integriert werden, die dazu ausgelegt ist, weitere Probenvorbereitungsschritte und/oder Probenanalyseschritte durchzuführen.

Bei Beispielen sind die Magnetelemente als magnetische Pole ausgeführt. Bei Beispielen sind die Magnetelemente dahingehend einzelne magnetische Pole, als dass die Lysevorrichtung Magnete außerhalb der Kammer aufweist, von denen nur jeweils ein magnetischer Pol einen signifikanten Einfluss auf den magnetischen Aktuator in der rotierenden Kammer hat, sodass der Einfluss weiterer Pole der Magneten außerhalb der Kammer, in der Regel der Einfluss des zweiten Pols eines jeden Magneten außerhalb der Kammer, für den magnetischen Aktuator in der Kammer vernachlässigt werden kann. Durch eine solche Anordnung kann der Aufbau spezieller Magnetfeldverläufe, welche für bestimmte Lyseanwendungen günstig sein können, ermöglicht werden. Bei Beispielen kann auch eine Verringerung des Bauraumbedarfs in radialer Richtung bezüglich der Drehebene der Kammer erzielt werden, wenn bspw. Stabmagnete senkrecht zur Drehebene angeordnet sind, sodass deren jeweiliger zweiter Pol ausreichend weit von der rotierenden Kammer entfernt ist, sodass deren jeweiliger Einfluss vernachlässigt werden kann. Bei Beispielen kann es sich bei den Magnetelementen um die Pole von gebogenen Magneten handeln, bei denen jeder Pol des gebogenen Magneten jeweils ein Magnetelement bildet. Der Rest des gebogenen Magneten, z.B. eines Hufeisenmagneten kann sich bspw. über oder unterhalb der Drehebene der Kammer befinden.

Bei Beispielen sind die Magnetelemente als Magnete ausgeführt. Bei solchen Beispielen kann ein besonders einfacher Aufbau möglich sein, da es z.B. keine Einschränkungen bezüglich des Einflusses eines zweiten Magnetpols, oder bspw. keinen zusätzlichen Bauraumbedarf durch gebogene Magnete, wie bei den Ausführungen der Magnetelemente als Magnetpole, gibt. Darüber hinaus ermöglichen solche Beispiele, bei denen jedes Magnetelement ein Magnet ist, den Aufbau spezieller Magnetfeldverläufe, welche für bestimmte Lyseanwendungen günstig sein können.

Bei Beispielen ist die Lysevorrichtung ausgelegt, um das magnetische Feld, das von den zumindest zwei außerhalb der Kammer angeordneten Magnetelementen auf den in der Kammer angeordneten magnetischen Aktuator wirkt, unabhängig von der rotatorischen Relativbewegung zumindest zu reduzieren. Dadurch ist es bspw. möglich, die Lyse zu beenden, ohne die rotatorische Relativbewegung zu beenden. Diese Art der Abschaltung kann bspw. vorteilhaft sein, wenn die rotatorische Relativbewegung für weitere Prozessschritte benötigt wird.

Bei Beispielen weist die Lysevorrichtung Betätigungsmittel auf, um den Abstand zwischen der Kammer und den zumindest zwei außerhalb der Kammer angeordneten Magnetelementen unabhängig von der rotatorischen Relativbewegung zu verändern. Dies kann beispielsweise durch eine translatorische Relativbewegung zwischen der Kammer und den außerhalb derselben angeordneten Magneten stattfinden. Dadurch ist es bspw. möglich, die Lyse durch eine einfache Translation der zumindest zwei außerhalb der Kammer angeordneten Magnetelemente zu beenden, ohne die rotatorische Relativbewegung zu beenden. Diese Art der Abschaltung ist bspw. einfach umzusetzen und kann bspw. vorteilhaft sein, wenn die rotatorische Relativbewegung für weitere Prozessschritte benötigt wird und daher nicht beendet werden soll.

Bei Beispielen weist die Lysevorrichtung Betätigungsmittel auf, die ausgelegt sind, um die zumindest zwei außerhalb der Kammer angeordneten Magnetelemente senkrecht zur Drehebene zu bewegen, sodass bspw. deren Einfluss auf die Lyse verringert oder verstärkt werden kann, sodass bspw. die Lyse beendet oder gestartet werden kann. Somit wird beispielsweise nur ein geringer Bauraum in radialer Richtung zur Umsetzung der Aktorik zur Bewegung der zumindest zwei Magnetelemente benötigt. Damit kann bspw. eine Gesamtintegration in eine Vorrichtung, welche dazu ausgelegt ist, weitere Probenvorbereitungs- und -analyseschritte durchzuführen, vereinfacht werden.

Bei Beispielen weist die Lysevorrichtung Betätigungsmittel auf, die ausgelegt sind, um die zumindest zwei außerhalb der Kammer angeordneten Magnetelemente parallel zur Drehebene unabhängig von der rotatorischen Relativbewegung zu bewegen, sodass bspw. die Lyse beendet werden kann, ohne die rotatorische Relativbewegung zu beenden. Bei solchen Beispielen kann für die Bewegung der zumindest zwei Magnetelemente von einer Rotationsachse weg die Fliehkraft genutzt werden, beispielsweise wenn die außerhalb der Kammer angeordneten Magnetelemente federnd gelagert sind und um dieselbe Rotationsachse rotieren. Beispielsweise kann durch die federnde Lagerung eine Kraft auf beide Magnetelemente ausgeübt werden, die zu der Rotationsachse hin wirkt, so dass durch Erhöhen der Rotationsgeschwindigkeit die Magnetelemente von der Rotationsachse entgegen der Federkraft von der Rotationsachse weg bewegt werden können. Auch diese Bewegung weg von der Rotationsachse und zu der Rotationsachse hin ist eine translatorische Bewegung unabhängig von der rotatorischen Relativbewegung.

Bei Beispielen können die zumindest zwei außerhalb der Kammer angeordneten Magnetelemente steuer- und/oder regelbare Elektromagnete sein, sodass bspw. deren Einfluss auf die Lyse verringert oder verstärkt werden kann. Bspw. kann ein Starten oder Beenden der Lyse unabhängig von der rotatorischen Relativbewegung durch die Elektromagneten bedingt werden, sodass weitere Prozesseschritte davor oder danach durchgeführt werden können, ohne weitere Aktorik zur Bewegung der zumindest zwei Magneten außerhalb der Kammer zu benötigen. Außerdem kann dadurch die Lysevorrichtung mit einem geringeren Bauraumbedarf ausgeführt werden.

Bei Beispielen weist die Lysevorrichtung zumindest einen und in der Regel mehrere Lysepartikel auf, die sich in der Kammer befinden, beispielsweise Mikropartikel, kugelförmige Mikropartikel oder Beads (Perlen). Der zumindest eine Lysepartikel kann bspw. aus Glas, Silika-Zirkonia, Zirkonia, Metall oder anderen Keramiken und Glasmaterialien bestehen. Bei Beispielen kann der zumindest eine Lysepartikel bspw. maximale Abmessungen von weniger als 0,5 mm aufweisen. Der zumindest eine Lysepartikel erleichtert dabei bspw. die Lyse der Probe, da durch zusätzliche Partikel die mechanischen Effekte auf die Probe in der Kammer verstärkt werden.

Bei Beispielen sind die zumindest zwei außerhalb der Kammer angeordneten Magnetelemente zum Zeitpunkt der Lyse stationär und die Antriebseinrichtung ist dazu ausgelegt, die Kammer bezüglich einer Drehachse relativ zu den außerhalb der Kammer angeordneten Magnetelementen zu rotieren. Dadurch kann bspw. ein sehr einfacher Aufbau der Lysevorrichtung und eine einfache Integration in eine Vorrichtung, die dazu ausgelegt ist, weitere Probenvorbereitungs- und -analyseschritte durchzuführen, erzielt werden, da nur die Kammer rotiert und keine rotatorische Aktorik für die zumindest zwei Magnetelemente außerhalb der Kammer notwendig ist.

Bei Beispielen befindet sich in der Kammer eine Membran, bspw. eine Filtermembran oder ein Sterilfilter, mit der eine Anreicherung von Mikroorganismen aus einem größeren Volumen und eine anschließende Lysierung auf der Membran ermöglicht wird.

Bei Beispielen weist die Lysevorrichtung eine Temperiereinrichtung auf, die ausgelegt ist, um die Temperatur der Kammer zu verändern, bspw. diese zu erhitzen, beispielsweise auf eine Temperatur von 120°C.

Die Temperiereinrichtung kann dabei als Kontaktheizung ausgeführt sein. Dadurch kann die mechanische Lyse durch einen thermischen Eintrag unterstützt werden.

Bei Beispielen sind die zumindest zwei Magnetelemente außerhalb der Kammer mit einem Winkel in der Rotationsebene zwischen 20° und 180° zueinander angeordnet. Der Winkel ist dabei zwischen Linien, die jeweilige Mittelpunkte der Magnetelemente mit der Rotationsachse verbinden, gebildet. Damit können geeignete Verläufe des magnetischen Feldes erzeugt werden, mit denen der magnetische Aktuator innerhalb der Kammer rotatorisch und translatorisch bewegt werden kann.

Bei Beispielen wird die rotatorische Relativbewegung in einem Drehfrequenzbereich von 0,5 Hz bis 40 Hz, vorzugsweise von 2 Hz bis 30 Hz durchgeführt. Durch diese Wahl des Frequenzbereichs kann bspw. eine effiziente Lyse mit ausreichend gutem und zugleich schnellem Ergebnis durchgeführt werden.

Bei Beispielen weist die Kammer in zwei von drei zueinander senkrechten Raumrichtungen mindestens die Länge der längsten Diagonale des magnetischen Aktuators, der sich innerhalb der Kammer befindet, auf und in einer dritten der drei zueinander senkrechten Raumrichtungen mindestens die Länge der längsten Diagonale des magnetischen Aktuators, der sich innerhalb der Kammer befindet, minus 20%. Bei Beispielen weist die Kammer in drei von drei zueinander senkrechten Raumrichtungen mindestens die Länge der längsten Diagonale des magnetischen Aktuators, der sich innerhalb der Kammer befindet, auf. Durch eine solche Dimensionierung der Kammer kann bspw. eine freie Bewegung des magnetischen Aktuators innerhalb der Kammer ermöglicht werden, oder bspw. eine definiert eingeschränkte Bewegung des magnetischen Aktuators, welche besonders günstig für die Lyse sein kann.

Bei Beispielen weist die Kammer in zumindest zwei Richtungen von drei Richtungen, die durch die Rotationsachsenrichtung, die radiale Richtung bezüglich der Rotation, und die azimutale Richtung bezüglich der Rotation gebildet sind, mindestens die Größe der Länge des magnetischen Aktuators, der sich innerhalb der Kammer befindet, auf. Durch eine solche Dimensionierung der Kammer kann bspw. eine bestimmte Art der Rotation ermöglicht werden, um bspw. eine effiziente Lyse zu ermöglichen. Diese Dimensionierung kann auch bspw. eine vorteilhafte Bauweise der Vorrichtung ermöglichen.

Bei Beispielen weisen die zumindest zwei Magnetelemente außerhalb der Kammer, zum Zeitpunkt der Lyse, einen maximalen senkrechten Abstand von 5 cm zur Kammer auf, in Bezug auf die Ebene der rotatorischen Relativbewegung. Bei Bespielen weisen die zumindest zwei Magnetelemente außerhalb der Kammer zum Zeitpunkt der Lyse einen maximalen radialen Abstand vom magnetischen Aktuator, der sich innerhalb der Kammer befindet, von 5 cm auf. Durch solche Abstände der Magnetelemente und des magnetischen Aktuators bzw. der Magnetelemente und der Kammer kann bspw. eine effiziente Lyse ermöglicht werden und zugleich bspw. nur ein geringer Bauraumbedarf für eine solche Vorrichtung notwendig sein.

Bei Beispielen weist die Lysevorrichtung eine Temperiereinrichtung auf, die ausgelegt ist den zumindest einen magnetischen Aktuator, der sich innerhalb der Kammer befindet, über seine Curie-Temperatur zu erhitzen, um den Magneten zu inaktivieren. Dadurch kann beispielsweise unabhängig von der rotatorischen Relativbewegung oder einer Aktorik für die zumindest zwei außerhalb der Kammer angeordneten Magnete oder deren möglichen Ausführung als Elektromagnete, eine Möglichkeit geschaffen werden, die Lyse zu beenden. Durch diese Möglichkeit kann bspw. eine besonders kleine Bauweise der Lysevorrichtung erzielt werden. Ebenfalls kann bspw. die Inaktivierung des magnetischen Aktuators innerhalb der Kammer für weitere Prozessschritte günstig sein.

Bei Beispielen werden die zumindest zwei außerhalb der Kammer angeordneten Magnetelemente, unabhängig von der rotatorischen Relativbewegung, bewegt, um das auf den in der Kammer angeordneten magnetischen Aktuator wirkende Magnetfeld zumindest zu reduzieren. Dadurch ist es bspw. möglich die Lyse zu beenden ohne die rotatorische Relativbewegung zu unterbrechen, welche bspw. für weitere Prozessschritte benötigt wird, sodass bspw. eine einfachere Integration in eine Prozesskette von Probenvorbereitung und -analyse möglich ist.

Bei Beispielen werden die zumindest zwei außerhalb der Kammer angeordneten steuerund/oder regelbare Elektromagneten so angesteuert oder geregelt, dass sich das auf den in der Kammer angeordneten magnetischen Aktuator wirkende Magnetfeld, unabhängig von der rotatorischen Relativbewegung, zumindest reduziert. Dadurch ist es bspw. möglich die Lyse zu beenden ohne die rotatorische Relativbewegung zu unterbrechen, welche bspw. für weitere Prozessschritte benötigt wird, sodass bspw. eine einfachere Integration in eine Prozesskette von Probenvorbereitung und -analyse möglich ist. Außerdem ist dabei auch bspw. keine weitere Aktorik zur Bewegung der zumindest zwei Magnetelemente außerhalb der Kammer zwingend notwendig. Dadurch kann bspw. die Lysevorrichtung mit einem geringeren Bauraumbedarf bei gleicher Funktionalität, im Vergleich zur Umsetzung mittels Permanentmagneten, ausgeführt werden.

Beispiele der vorliegenden Offenbarung beziehen sich auf ein Verfahren zur effizienten Lyse von Mikroorganismen aus komplexen Proben.

Bei Beispielen befinden sich kugelförmige Mikropartikel, ein magnetischer Aktuator und die Probe in einer Lysekammer einer zentrifugal mikrofluidischen Kartusche. Mindestens zwei Permanent- oder Elektromagnete sind z.B. über oder unter der Kartusche bspw. statisch angeordnet. Durch die Rotation der Kartusche kann in der Lysekammer ein sich ständig wechselndes Magnetfeld erzeugt werden. Der Aktuator kann dadurch stark in Bewegung versetzt werden, er kann sich sowohl translatorisch, zum nächstgelegenen Magneten, als auch rotatorisch um zumindest eine eigene Achse bewegen. Im Zusammenspiel mit Mikropartikeln kommt kann es zu starken Reibe-, Stoß- und Scherbewegungen in der Lysekammer kommen.

Um gleichzeitig eine Translation als auch Rotation des magnetischen Aktuators zu erzielen, kann die Lysekammer dazu ausgelegt sein, in zwei Raumrichtungen mindestens die Größe der Länge des magnetischen Aktuators aufzuweisen. Des Weiteren weisen die mindestens zwei externen, also außerhalb der Lysekammer befindlichen, Magnetelemente einen Winkel zueinander auf und sind relativ zur Lysekammer mit entgegengesetzter Polung angeordnet.

Beispiele der vorliegenden Offenbarung sind damit dazu ausgelegt, Bakterien, Hefen, Vieren, Pilze, Sporen oder andere Mikroorganismen in der Probe effizient in kürzester Zeit aufzuschließen, also zu lysieren.

Beispiele der vorliegenden Offenbarung beziehen sich auf ein Verfahren zur mechanischen Lyse auf einer zentrifugal mikrofluidischen Struktur. Beispiele ermöglichen es, bei minimalem Handhabungsaufwand, eine effiziente Lyse bereitstellen zu können, bei der selbst schwer-zu-lysierende Mikroorganismen bewältigt werden können. Als einer der Kernschritte in der Probenvorbereitung ermöglicht eine effiziente Lyse einen hoch sensitiven, molekular diagnostischen Nachweis z.B. mittels qPCR (quantitative polymerase chain reaction = quantitative Polymerase-Kettenreaktion).

Bei Beispielen werden Rotations- und Translationsbewegungen eines magnetischen Aktuators durch Rotation eines Fluidikmoduls bzw. einer Kartusche durch ein externes statisches Magnetfeld bewirkt.

Weitere Beispiele umfassen Lysevorrichtungen, bei denen die Polung der Magnetelemente außerhalb der Kammer, bspw. der externen Magnete entgegengesetzt zueinander in Bezug auf die Lysekammer ist, um eine Rotationbewegung des magnetischen Aktuators zu initiieren.

### Kurze Beschreibung der Zeichnungen

Beispiele der vorliegenden Offenbarung werden nachfolgend Bezug nehmend auf die beiliegenden Zeichnungen näher erläutert. Es zeigen:
- Fig. 1a: eine schematische Aufsicht eines Beispiels einer Lysevorrichtung;
- Fig. 1b: eine schematische Seitenansicht der Lysevorrichtung von Fig. 1a;
- Fig. 2: eine schematische Aufsicht eines weiteren Beispiels einer Lysevorrichtung, bei dem Magnete in einem anderen Winkel angeordnet sind;
- Fig. 3: eine schematische Aufsicht eines weiteren Beispiels einer Lysevorrichtung mit einer anderen Anordnung der Magnete;
- Fig. 4: eine schematische Aufsicht eines Beispiels einer Lysevorrichtung mit einer weiteren unterschiedlichen Anordnung von Magnetelementen;
- Fig. 5: schematische Seitenansichten zweier Beispiele einer Lysevorrichtung, mit Betätigungsmitteln zur Beeinflussung des magnetischen Feldes auf den magnetischen Aktuator, unabhängig von der rotatorischen Relativbewegung;
- Fig. 6: eine Auftragung zum Vergleich einer qPCR-Analyse eines Lysats entsprechend der vorliegenden Offenbarung und eines thermischen Referenzlysats;
- Fig. 7: eine schematische Seitenansicht eines Beispiels einer Kammer einer Lysevorrichtung mit einer Membran;
- Fig. 8: eine schematische Seitenansicht eines Beispiels einer Lysevorrichtung mit einer Temperiereinrichtung.

### Detaillierte Beschreibung

Im Folgenden werden Beispiele der vorliegenden Offenbarung detailliert und unter Verwendung der beigefügten Zeichnungen beschrieben. Es sei darauf hingewiesen, dass gleiche Elemente oder Elemente, die die gleiche Funktionalität aufweisen, mit gleichen oder ähnlichen Bezugszeichen versehen sind, wobei eine wiederholte Beschreibung von Elementen, die mit dem gleichen oder ähnlichen Bezugszeichen versehen sind, typischerweise weggelassen wird. Insbesondere können gleiche oder ähnliche Elemente jeweils mit Bezugszeichen versehen sein, die eine gleiche Zahl mit einem unterschiedlichen oder keinem Kleinbuchstaben aufweisen. Beschreibungen von Elementen, die gleiche oder ähnliche Bezugszeichen aufweisen, sind gegeneinander austauschbar. In der folgenden Beschreibung werden viele Details beschrieben, um eine gründlichere Erklärung von Beispielen der Offenbarung zu liefern. Es ist jedoch für Fachleute offensichtlich, dass andere Beispiele ohne diese spezifischen Details implementiert werden können. Merkmale der unterschiedlichen beschriebenen Beispiele können miteinander kombiniert werden, es sei denn Merkmale einer entsprechenden Kombination schießen sich gegenseitig aus oder eine solche Kombination ist ausdrücklich ausgeschlossen.

Bevor Beispiele der vorliegenden Offenbarung näher erläutert werden, werden Definitionen, einiger hierin verwendeter Begriffe angegeben.

Kartusche: Kartuschen sind Polymer-Einwegteile, die Kanäle, Kammern zur Führung, Aufbereitung und Analyse von Proben beinhalten. Des Weiteren können Kartuschen Schnittstellen zum Probeneintrag und potentiell der Entnahme von Flüssigkeiten enthalten.

Probe: Eingebrachte Substanz (oft eine Flüssigkeit), die Mikroorgansimen enthält.

Lyse: Zerstörung einer Zelle durch Schädigung der äußeren Zellmembran.

Mikropartikel / Beads (Perlen) als Beispiele von Lysepartikeln: Kugelförmige Elemente mit einem typischen Durchmesser von 0,1 mm - 3 mm aus z.B. Glas, Silika-Zirkonia, Zirkonia, Metall oder andere Keramiken und Glasmaterialien.

Magnetischer Aktuator: Kleiner mitrotierender Magnet z.B. Stabmagnet, der durch ein externes Magnetfeld in Bewegung gebracht wird.

Lysekammer: Kammer bspw. auf Kartusche in welcher der Prozess der Lyse durchgeführt wird, sie beinhaltet bspw. Partikel und zumindest einen magnetischen Aktuator.

Externer Magnet: Permanent - oder Elektromagnet, der bspw. außerhalb der Kammer, z.B. über oder unter der Kartusche bspw. statisch angeordnet sein kann.

Polung von zwei Magneten: Ausrichtung von Nord- und Südpol in Bezug auf die (Δr, Δϕ, bspw. radial und azimutal bezüglich eines Rotationszentrums) - Ausdehnung der Lysekammer, bzw. in Bezug auf die Kreisbahn einer rotatorischen Relativbewegung zwischen der Kammer und den Magnetelementen außerhalb der Kammer.

Rotatorische Bewegung: Beschreibt die Bewegung eines magnetischen Aktuators, um die eigene Achse.

Translatorische Bewegung: Beschreibt dieselbe Verschiebung aller Punkte eines starren Körpers, z.B. eines magnetischen Aktuators, zu einem gegebenen Zeitpunkt. Geschwindigkeit und Beschleunigung aller Punkte sind identisch und sie bewegen sich auf parallelen Trajektorien.

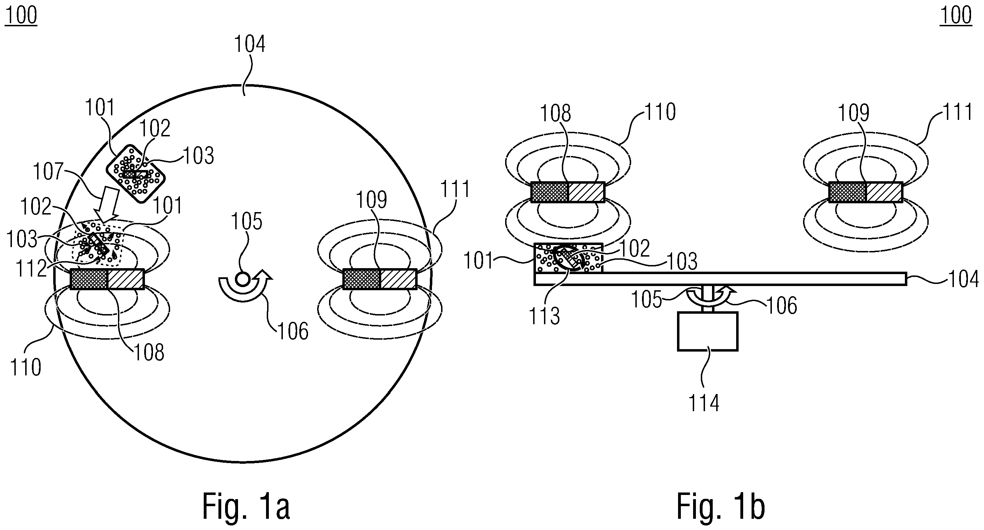

Fig. 1a zeigt eine schematische Aufsicht und Fig. 1b eine schematische Seitenansicht eines Beispiels einer Lysevorrichtung. Die dargestellte Lysevorrichtung 100 besteht aus einer Kammer 101, die einen magnetischen Aktuator 102 und Lysepartikel 103 umfasst. Der magnetische Aktuator 102 ist bei dem gezeigten Beispiel ein Stabmagnet. Die Kammer ist auf einem Träger 104 befestigt. Der Träger 104 und damit auch die Kammer 101 sind dazu ausgelegt, um eine Rotationsachse 105 zu rotieren. Die rotatorische Bewegung des Trägers ist mit einem Pfeil 106 angedeutet. Die daraus resultierende rotatorische Bewegung der Kammer auf einer Kreisbahn ist mit einem weiteren Pfeil 107 angedeutet. Bei diesem Beispiel befinden sich oberhalb der Kammer, also senkrecht zur Rotationsebene, zwei statische Magnetelemente 108, 109, die als Magnete ausgeführt sind. Das Magnetfeld des Magnetelementes 108 ist durch einen Feldlinienverlauf 110, das Magnetfeld des Magnetelementes 109 durch den Feldlinienverlauf 111 angedeutet. Die Magnetelemente 108, 109 sind bezüglich der Verbindungslinie ihrer jeweiligen Nord- und Südpole (magnetische Achse) orthogonal zur rotatorischen Kreisbahn der Kammer ausgerichtet. Darüber hinaus haben die beiden Magnetelemente 108, 109 bezüglich der rotatorischen Kreisbahn der Kammer eine entgegengesetzte Polung. Auf die entgegengesetzte Polung wird dabei im Zuge der folgenden Beschreibung der Funktionalität näher eingegangen. Die Magnetelemente sind bezüglich der Kreisbahn in einem Winkel von 180° zueinander angeordnet. Die Kammer ist in gepunkteten Linien zu einem zweiten Zeitpunkt dargestellt. Dies zeigt die Bewegung der Kammer 101 auf der rotatorischen Kreisbahn um die Rotationsachse 105. Innerhalb der bewegten Kammer 101 ist der sich bewegende magnetische Aktuator 102 ebenfalls zu dem zweiten Zeitpunkt in veränderter Lage und Orientierung im Vergleich zum magnetischen Aktuator vor der Bewegung dargestellt. Die bewegten Lysepartikel 103 zum zweiten Zeitpunkt sind in der bewegten Kammer 101 ebenfalls dargestellt. Die Bewegung der Lysepartikel selbst, auf Grund der Rotation der Kammer 101, sowie auf Grund der Bewegung des magnetischen Aktuators 102 ist mit Pfeilen 112 angedeutet. Ferner ist die rotatorische Bewegung des magnetischen Aktuators während der Rotation der Kammer mit Pfeilen 113 in Fig. 1b angedeutet. Zum Bewirken der rotatorischen Bewegung des Trägers 104 ist eine Antriebseinrichtung 114 an der Rotationsachse 105 dargestellt.

Bei Beispielen rotiert die Kammer 101 im Betrieb auf einer rotatorischen Kreisbahn bezüglich der Rotationsachse 105. Im Laufe der Rotation nähert sich die bewegte Kammer 101 dem Magnetelement 108. Durch das magnetische Feld des Magnetelementes 110 kommt es zu einer Wechselwirkung mit dem magnetischen Aktuator 102 innerhalb der Kammer. Der magnetische Aktuator 102 erfährt eine translatorische Bewegung auf Grund der magnetischen Anziehung und richtet sich durch eine Drehbewegung entsprechend dem Feld 110 des Magnetelementes 108 aus. Durch die Rotation der Kammer 101 bezüglich der Rotationsachse 105 hat der magnetische Aktuator 102 innerhalb der Kammer allerdings nur eine kurze Verweildauer in unmittelbarer Nähe des ersten Magnetelementes 108. Durch die Rotation wird sich die Kammer dem nächsten Magnetelement 109 nähern. Die Polung des Magnetelementes 109 ist dabei in Bezug auf die rotatorische Kreisbahn der Kammer entgegengesetzt zu dem zuvor durch die Kammer 101 passierten Magnetelement 108.

In einfachen Worten: Rotiert man gedanklich mit der Kammer 101 auf der Kreisbahn mit, so ist in Rotationsrichtung in Bezug auf die Kammer kurz vor dem Passieren des ersten Magnetelementes 108 rechtsseitig der Nordpol und linksseitig der Südpol des Magnetelementes 108. Beim Weiterrotieren trifft die Kammer 101 dann auf das zweite Magnetelement 109. In Richtung ihrer Rotation ist aus Sicht der Kammer hier rechtsseitig der Südpol und linksseitig der Nordpol des Magnetelementes 109.

Dementsprechend erfährt ein magnetischer Aktuator 102, der sich innerhalb der Kammer 101 befindet, im Laufe der Rotation der Kammer in Bezug auf die Kreisbahn der Kammer, entgegengesetzt orientierte Magnetfelder 110, 111 von jeweils aufeinander folgenden entgegengesetzt gepolten Magnetelementen 108, 109. Die zuvor beschriebene Drehbewegung des magnetischen Aktuators 102 führt durch die aufeinanderfolgenden, bezüglich der rotatorischen Kreisbahn der Kammer, gegenpoligen Magnetelemente 108, 109, zu einer Rotation 113 des Aktuators 102, innerhalb der Kammer. Die Rotation wird dabei von der translatorischen Bewegung und der Trägheit des magnetischen Aktuators mitbeeinflusst. In anderen Worten: Der magnetische Aktuator 102 führt eine translatorische Bewegung innerhalb der Kammer aufgrund der magnetischen Anziehung zu den Magnetelementen 108, 109 aus und zusätzlich rotiert 113 der magnetische Aktuator 102 innerhalb der Kammer aufgrund der rotatorischen Relativbewegung zwischen der Kammer 101 und den bezüglich der rotatorischen Kreisbahn der Kammer entgegengesetzt gepolten Magnetelementen 108, 109.

Durch die Rotation 113 und Translation des magnetischen Aktuators 102 wird die Probe, insbesondere mit Hilfe der durch den magnetischen Aktuator in Bewegung versetzten 112 Lysepartikeln 103 lysiert.

Bei dem Träger 104 kann es sich dabei um einen zentrifugal-mikrofluidischen Testträger, wie er beispielsweise unter der Bezeichnung LabDisk oder LabDisk Struktur bekannt ist, oder um eine Kartusche oder ein Fluidikmodul handeln. Ein solcher Träger umfasst dabei bspw. eine Lysekammer, in der sich ein magnetischer Aktuator, Partikel sowie die Probe befinden. Über dem Träger können z.B. zwei ortsfeste, relativ zur Lysekammer entgegengesetzt gepolte Magnete positioniert sein. Durch die Rotation eines Trägers, bspw. einer Kartusche kann dabei der magnetische Aktuator in Rotation und Translation gebracht werden, die Partikel können dadurch stark durchmischt und bspw. Bakterien in der Probe lysiert werden.

Bei Beispielen kann die Kammer 101 auch Teil des Trägers 104 sein, oder bspw. in dem Träger integriert sein. Der magnetische Aktuator 102 kann bspw. als Stabmagnet ausgeführt sein. Die Magnetelemente 108, 109 können als statische Magnetelemente ausgeführt sein, so dass lediglich die Kammer 101 auf einer Kreisbahn bzgl. einer Rotationsachse 105 rotiert. Allerdings ist es ebenfalls möglich, dass sowohl die Kammer 101 als auch die Magnetelemente 108, 109 bezüglich derselben Rotationsachse 105 rotieren, oder dass die Kammer statisch ist und nur die Magnetelemente 108, 109 bezüglich einer Rotationsachse 105 rotieren. Dementsprechend ist die Antriebseinrichtung 114 auch nicht nur als exklusiver Antrieb der Rotation der Kammer 101 zu verstehen. Eine entsprechende Nutzung der Antriebseinrichtung zum Einbringen einer Rotation der zumindest zwei Magnetelemente 108, 109 außerhalb der Kammer um die gleiche Rotationsachse 105 ist ebenfalls zusätzlich, oder alleinig möglich.

Alternativ können die Magnetelemente 108, 109 auch unterhalb der Kammer 101, oder neben dem Träger 104 und damit der Kammer 101, bspw. außerhalb des Kreises der rotatorischen Bewegung der Kammer 101, angeordnet sein. Die Magnetelemente außerhalb der Kammer können dabei bspw. als Permanent-, oder Elektromagnete, oder als einzelne Pole der Magnete ausgeführt sein. Darüber hinaus können auch mehr als zwei Magnetelemente außerhalb der Kammer angeordnet sein. Die entgegengesetzte Polung der Magnetelementen ist dann für jeweils zwei, bezüglich der rotatorischen Relativbewegung der Kammer, in Bewegungsrichtung der Kammer, aufeinanderfolgenden Magnetelemente zu verstehen. Darüber kann sich bei Beispielen mehr als ein magnetischer Aktuator in der Kammer befinden.

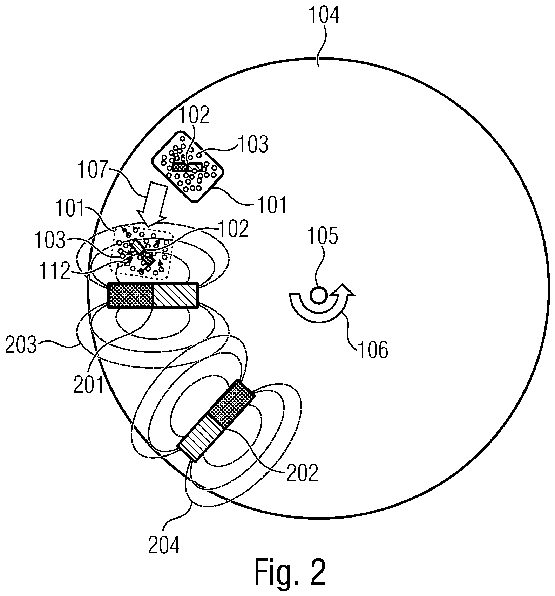

Fig 2 zeigt eine schematische Aufsicht eines Beispiels einer Lysevorrichtung, bei der die Magnetelemente in einem anderen Winkel angeordnet sind.

Die Darstellung entspricht Fig. 1a, mit Ausnahme der Anordnung der Magnetelemente in Form von Stabmagneten außerhalb der Kammer bezüglich ihres Winkels zueinander. Gleiche Elemente sind daher mit gleichen Bezugszeichen versehen. Das Beispiel umfasst zwei Magnetelemente 201, 202, deren Magnetfelder mit magnetischen Feldlinien 203 für das Magnetelement 201, und magnetischen Feldlinien 204 für das Magnetelement 202 angedeutet sind. Die beiden Magnetelemente 201, 202 sind im Vergleich zu Fig. 1a in einer anderen Winkelanordnung zueinander dargestellt. Der Winkel ist dabei zwischen Linien, die jeweilige Mittelpunkte der Magnetelemente mit der Rotationsachse verbinden, gebildet. Bei dem gezeigten Beispiel beträgt der Winkel ca. 55°. Es hat sich gezeigt, dass eine effektive Lyse erreicht werden kann, wenn der Winkel in einem Winkelbereich von 20° bis 180° liegt.

Die Funktionsweise ist dabei analog zu Fig. 1a. Durch die Rotation des Trägers 104 passiert die auf dem Träger 104 befestigte Kammer 101 das erste der beiden Magnetelemente 201, sodass bezüglich der Kreisbahn der Rotation der Kammer, in Bewegungsrichtung der Kammer, der Nordpol des ersten Magnetelements von der Kammer 101 aus rechts liegt und der Südpol links, und beim Passieren des zweiten Magnetelementes ist dessen Polung entgegengesetzt zum ersten Magnetelement, sprich der Nordpol des zweiten Magnetelementes 202 liegt bezüglich der Kreisbahn der Rotation der Kammer, in Bewegungsrichtung der Kammer von der Kammer 101 aus entsprechend links und der Südpol rechts. Dadurch erfährt der magnetische Aktuator eine Rotation und eine Translation, sodass eine effektive Lyse der Probe möglich ist.

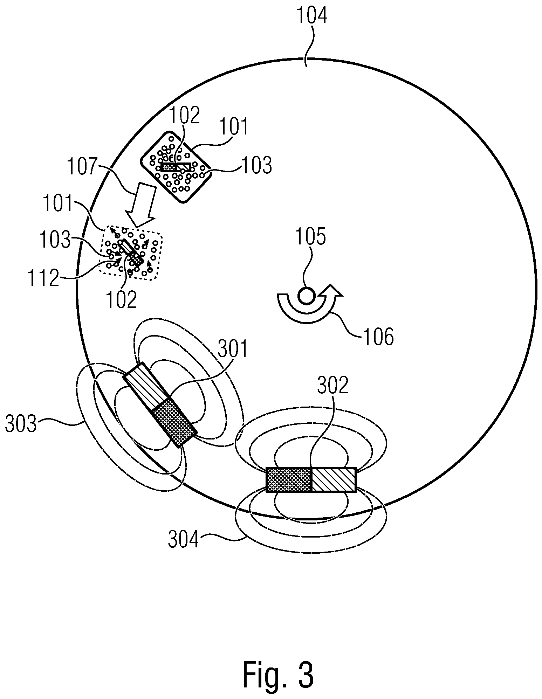

Fig. 3 zeigt eine schematische Aufsicht eines Beispiels einer Lysevorrichtung, bei der die Magnetelemente in Form von Stabmagneten bezüglich der Kreisbahn eine andere Ausrichtung aufweisen. Die Darstellung entspricht Fig. 1a, mit Ausnahme der Anordnung der Magnetelemente außerhalb der Kammer. Gleiche Elemente sind daher mit gleichen Bezugszeichen versehen. Das Beispiel umfasst zwei Magnetelemente 301, 302, deren Magnetfelder mit magnetischen Feldlinien 303 für das Magnetelement 301, und magnetischen Feldlinien 304 für das Magnetelement 302 angedeutet sind. Die Magnetelemente 301, 302 weisen im Vergleich zu Fig. 1a sowohl eine andere Winkelanordnung auf, als auch eine andere Lage bezüglich der Kreisbahn. Die Magnetelemente 301, 302 sind im Vergleich zu Fig. 1a, zusätzlich zur veränderten Winkelanordnung, um 90° verdreht dargestellt, sodass die Verbindungslinie zwischen Nord- und Südpol eines Magnetelementes (magnetische Achse) tangential zur rotatorischen Kreisbahn der Kammer liegt.

Fig. 3 soll damit eine weitere mögliche Ausführungsform der entgegengesetzt gepolten Magnetelemente verdeutlichen. Durch die Rotation des Trägers 104 passiert die auf dem Träger befestigte Kammer 101 das erste Magnetelement 301. Bezüglich ihrer Kreisbahn trifft die Kammer 101 durch die Rotation im Hinblick auf das erste Magnetelement 301 zuerst auf dessen Südpol und anschließend auf dessen Nordpol. Beim Passieren des zweiten Magnetelementes 302, das außerhalb der Kammer angeordnet ist, ist die Polung entgegengesetzt zur Polung des ersten Magnetelementes 301. Bezüglich ihrer Kreisbahn trifft die Kammer 101 durch die Rotation im Hinblick auf das zweite Magnetelement 302 nun zuerst auf dessen Nordpol und anschließend auf dessen Südpol, also genau entgegengesetzt zum ersten Magnetelement 301. Der magnetische Aktuator 102 innerhalb der Kammer erfährt durch die magnetische Anziehung zu den Magnetelementen 301, 302 eine translatorische Bewegung. Beim Passieren des ersten Magnetelementes, durch die Rotation der Kammer, wird sich der Aktuator 102 entsprechend des magnetischen Feldes des ersten Magnetelementes 303 durch eine Drehung ausrichten. Durch die Rotation der Kammer kommt der magnetische Aktuator darauffolgend in den unmittelbaren Wirkungsbereich des zweiten Magnetelements 302, dessen Feld 304 den magnetischen Aktuator 102 wiederum durch eine Drehung zur Ausrichtung entsprechend seines bezüglich der Kreisbahn der Kammer entgegengesetzt zum Feld 303 des ersten Magnetelementes orientierten, magnetischen Feldes 304 zwingen wird. Daher erfährt der magnetische Aktuator 102 durch die Rotation der Kammer 101 und der entgegengesetzten Polung der Magnetelemente 301, 302 außerhalb der Kammer eine Rotation zusätzlich zur translatorischen Bewegung aufgrund der magnetischen Anziehung zu den Magnetelementen 301, 302.

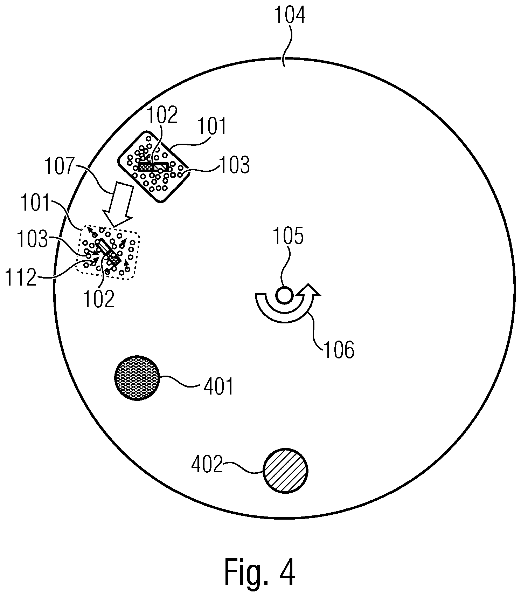

Fig. 4 zeigt eine schematische Aufsicht eines Beispiels einer Ausführungsform der Magnetelemente der Lysevorrichtung. Die Darstellung entspricht Fig. 1a, mit Ausnahme der Anordnung und der Art der Magnetelemente außerhalb der Kammer. Gleiche Elemente sind daher mit gleichen Bezugszeichen versehen. Das Beispiel umfasst zwei Magnetelemente 401, 402, die im Vergleich zu Fig. 1a eine andere Winkelanordnung aufweisen und darüber hinaus nur einzelne magnetische Pole darstellen. Das Magnetelement 401 stellt einen Nordpol dar, das Magnetelement 402 stellt einen Südpol dar.

Fig. 4 soll damit eine weitere mögliche Ausführungsform der entgegengesetzt gepolten Magnetelemente verdeutlichen. Durch die Rotation des Trägers 104 trifft die auf dem Träger befestigte Kammer 101 auf das erste Magnetelement 401, das einen Nordpol darstellt. Der magnetische Aktuator 102 erfährt eine Translation aufgrund der magnetischen Anziehung und eine Drehbewegung, sodass sich der Südpol des Aktuators in Richtung des Magnetelementes ausrichtet. Nach dem Passieren des Magnetelementes 401 liegt in Bezug auf die Bewegungsrichtung der Kammer, das Magnetelement 401, auf den sich der Südpol des Aktuators ausgerichtet hat, hinter und nicht mehr vor der Kammer. Dementsprechend wird sich der magnetische Aktuator 102 drehen, sodass wiederum der Südpol des magnetischen Aktuators zu dem Magnetelement 401 ausgerichtet ist. Bei der weiteren Rotation der Kammer trifft diese auf das zweite Magnetelement 402, das einem Südpol darstellt, entgegengesetzt zur Polung des ersten Magnetelementes 401. Durch das Vorbeirotieren am zweiten Magnetelement 402 wird sich in diesem Fall der Nordpol des magnetischen Aktuators auf das zweite Magnetelement ausrichten, wodurch der magnetische Aktuator 102 wiederum eine Drehbewegung erfährt. Durch die Rotation der Kammer 101 erfährt der magnetische Aktuator 102 neben der Translation durch die magnetische Anziehung eine Abfolge von Drehbewegungen, sodass er rotiert und damit die Lyse der Probe unterstützt.

Eine solche Anordnung von Magnetelementen 401, 402 kann bspw. aus Magneten bestehen, deren jeweils zweiter Pol so weit von der Kammer entfernt ist, dass dieser jeweils zweite Pol bezüglich seines Einflusses auf den magnetischen Aktuator vernachlässigt werden kann. Eine weitere Möglichkeit wäre die Verwendung von gebogenen Magneten, bspw. in der Form von Hufeisenmagneten, sodass die beiden Pole eines jeden Magneten außerhalb der Kammer bezüglich ihres Einflusses auf die Kammer näherungsweise als magnetische Einpole in der Rotationsebenen angenähert werden können. Bei dieser Umsetzung könnte sich der Rest der gebogenen Magnete bspw. über oder unter der Anordnung befinden. Eine andere Möglichkeit eines solchen Aufbaus besteht in der Ausführung der Magnetelementen 401, 402 als Stabmagnete, wobei die Verbindungslinie zwischen Nordpol und Südpol senkrecht zur Rotationebene ist, so dass in Fig. 4 jeweils nur ein Pol gezeigt ist.

Diese möglichen Anordnungen der externen Magnetelemente oder bspw. Magneten mit relativ zur Kammer, bspw. Lysekammer unterschiedlicher Polung sind dabei nur als Beispiele zur Verdeutlichung einiger Aspekte der Offenbarung aufzufassen und keinesfalls eine abschließende Aufzählung. Insbesondere sind Anordnungen mit mehr als zwei Magnetelementen, sowie verschiedenste Winkelanordnungen der zumindest zwei Magnetelemente, sowie weitere Lageausrichtungen der Magnetelemente bezüglich der rotatorischen Kreisbahn ebenfalls Teil der vorliegenden Offenbarung im Sinne einer offensichtlichen Abwandlung durch einen Fachmann. Insbesondere sollen die Darstellungen keine Einschränkung bezüglich der Form der Magnetelemente darstellen. Die Magnetelemente, können beispielsweise runde, quadratische oder rechteckige Querschnitte haben. Das Gleiche gilt für die Formgebung des magnetischen Aktuators.

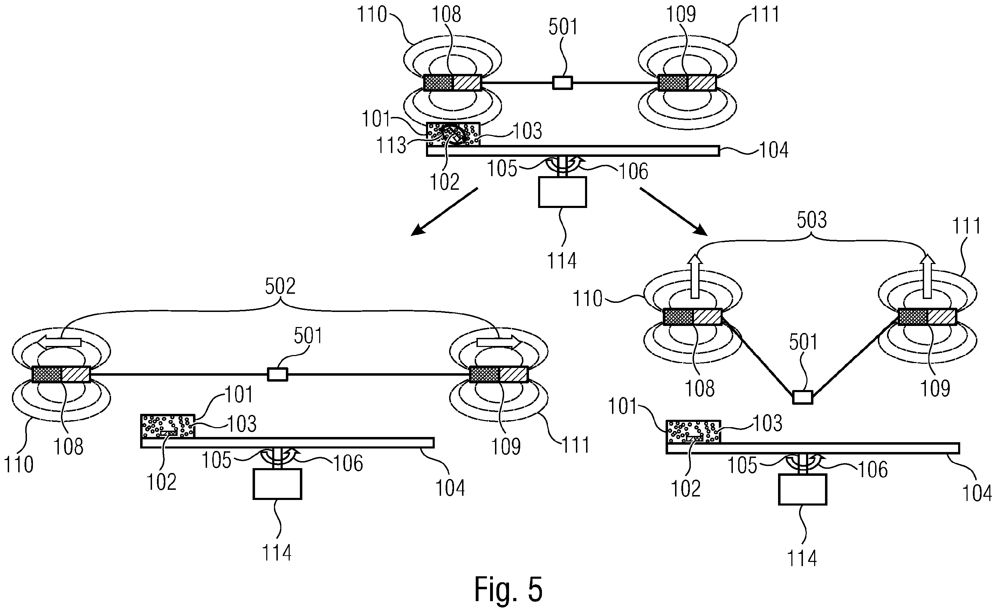

Fig. 5 zeigt schematische Seitenansichten zweier Beispiele der Lysevorrichtung, mit Betätigungsmitteln zur Beeinflussung des magnetischen Feldes auf den magnetischen Aktuator, unabhängig von der rotatorischen Relativbewegung.

Die Darstellungen entsprechen Fig. 1b, mit Ausnahme des Betätigungsmittels und der Lage der Magnetelemente außerhalb der Kammer bei einer zusätzlichen translatorischen Bewegung unabhängig von der rotatorischen Relativbewegung zwischen der Kammer und den Magnetelementen. Gleiche Elemente sind daher mit gleichen Bezugszeichen versehen.

Fig. 5 umfasst Betätigungsmittel 501, die mit den Magnetelementen 108, 109 verbunden sind. Die Ausgangsanordnung ist in Fig. 5 oben dargestellt. Fig. 5 unten links zeigt eine Möglichkeit der translatorischen Bewegung 502 der Magnetelemente parallel zur Rotationsebene. Fig. 5 unten rechts zeigt eine Möglichkeit der translatorischen Bewegung 503 der Magnetelemente orthogonal zur Rotationsebene.

Durch Betätigungsmittel 501 können die Magnetelemente 108, 109 translatorisch, unabhängig von der rotatorischen Relativbewegung zwischen der Kammer 101 und den Magnetelementen 108, 109, bewegt werden. Fig. 5 unten links zeigt ein Bewegen 502 der Magnetelemente 108, 109 parallel zur Rotationsebene. Fig. 5 unten rechts zeigt ein Bewegen 503 der Magnetelemente 108, 109, orthogonal zur Rotationsebene. Durch beide Varianten können die Magnetelemente so weit von der rotierenden Kammer wegbewegt werden, dass die magnetische Wechselwirkung zwischen den Magnetelementen 108, 109 und dem magnetischen Aktuator derart abgeschwächt wird, dass die Lyse zum Erliegen kommt.

Durch die translatorische Bewegung der Magnetelemente unabhängig von der rotatorischen Bewegung der Kammer kann die Lyse gestoppt oder aber auch bspw. gestartet, verstärkt oder abgeschwächt werden. Durch die Betätigungsmittel 501 ist eine Beeinflussung der Lyse möglich, sodass mit einer solchen Anordnung auch bspw. weitere Schritte einer Probenvorbereitung oder Probenanalyse durchgeführt werden können, für die bspw. eine Rotation der Kammer notwendig ist, aber eine Lyse nicht stattfinden soll. Darüber hinaus sind auch weitere translatorische Bewegungsrichtungen der Magnetelemente denkbar, z.B. ein Wegbewegen der Magnetelemente in einem bestimmten Winkelbereich, bspw. in einem Winkel, der zwischen den dargestellten Bewegungsrichtungen, also zwischen orthogonal und parallel zur Rotationsebene, liegt.

In anderen Worten liegt Beispielen der Offenbarung die Idee zugrunde, dass durch die Rotation eines Trägers, bspw. einer Disk (Scheibe), ein magnetischer Aktuator ein wechselndes Magnetfeld erfährt, sobald er unter einem der externen Magnetelemente, bspw. unter statischen Magneten, durchfährt. Durch die unterschiedliche Polung der externen Magnetelemente erfährt der magnetische Aktuator eine Translations- und Rotationsbewegung. Dadurch können Lysepartikel miteinander kollidieren und dabei durch Reibe-, Stoß- und Scherkräfte Mikroorganismen (bspw. Viren, Bakterien, Pilze, Parasiten) lysieren.

Bei Beispielen können dann, nachdem die Mikroorganismen lysiert sind, die externen bspw. statischen Magnete über ein Wegfahren einer Halterung (bspw. entsprechend Fig. 5) von der Kammer, bspw. Lysekammer und dem magnetischen Aktuator, bspw. Stabmagneten entfernt werden. Dadurch überwiegt die Zentrifugal- und Schwerkraft auf dem magnetischen Aktuator, was die starke Translation und Rotation und damit die Lyse stoppt.

Die vorliegende Offenbarung umfasst dabei insbesondere die örtliche Trennung der externen, bspw. statischen Magnetelemente vom magnetischen Aktuator in der Lysekammer. Beispiele umfassen dabei eine örtliche Trennung durch seitliche Translation (bspw. Fig. 5 links unten) oder örtliche Trennung durch vertikale Translation (bspw. Fig. 5 rechts unten).

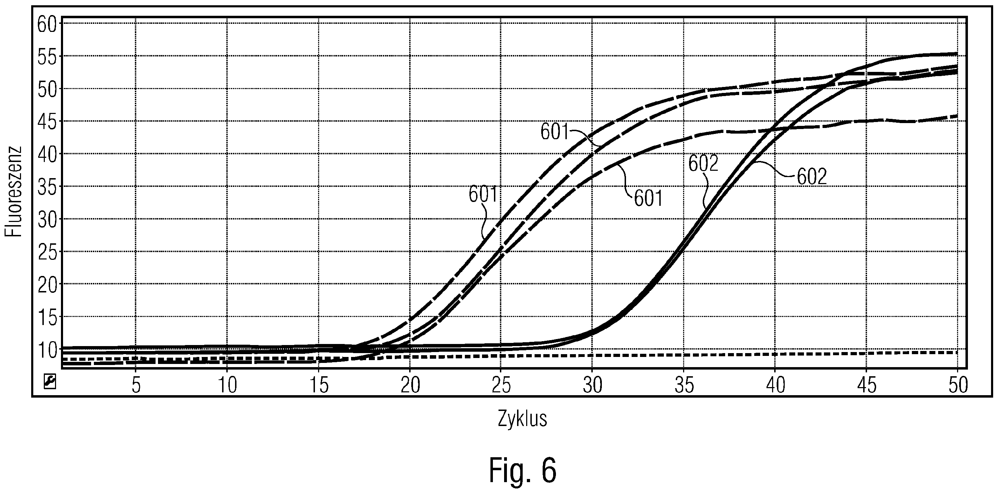

Fig. 6 zeigt eine Auftragung zum Vergleich einer qPCR-Analyse eines durch eine Lyse entsprechend der vorliegenden Offenbarung erhaltenen Lysats, und eines thermischen Referenzlysats. Dabei ist auf der Ordinate ein Maß für das Ergebnis der Lyse, bspw. die Fluoreszenz, auf der Abszisse ein Maß für die Zeit, bspw. die Zyklen, aufgetragen. Die Auftragung der qPCR-Analyse 601 eines Lysat entsprechend der vorliegenden Offenbarung zeigt dabei einen sehr viel früheren Anstieg der Kurve bzgl. der Ordinate. Die Auftragung der qPCR-Analyse 602 des thermischen Referenzlysats zeigt einen deutlich späteren Anstieg des Maßes für die Effektivität der Lyse. Die Auftragungen weisen ein Abflachen der Kurve nach ihrem jeweiligen Anstieg an. Die Absolutwerte bzgl. der Lyseeffektivität unterscheiden sich in diesen Bereichen der Auftragungen für beide Herangehensweisen nur gering.

Die mechanische Lyse entsprechend der vorliegenden Offenbarung zeigt demnach eine vielfach höhere Lyseeffizienz als die thermische Referenzlyse. Eine Steigerung der Lyseeffektivität ist dabei allerdings auch durch eine geeignete Vorgehensweise entsprechend der vorliegenden Offenbarung möglich.

Die Ergebnisse nach Fig. 6 wurden dabei unter Verwendung eines Versuchs mit einem Aufbau auf einem mikrofluidischen Testsystem erzielt, das als Träger eine Scheibe, die unter der Bezeichnung LabDisk-Foliendisk bekannt ist, und zwei Permanentmagneten (bspw. Neodym, Höhe 5mm; Durchmesser 15 mm; Stärke der Magnetisierung 45SH; Art der Beschichtung: Nickel (Ni-Cu-Ni)) auf einem Radius von 55 mm, etwa 8 mm unterhalb des Trägers aufwies.

Dabei wurden Glaspartikel (0,1 mm Durchmesser und 0,5 mm Durchmesser) und ein magnetischer Aktuator, bspw. Stabmagnet (2 mm Durchmesser und 3mm Höhe, Magnetisierung N45, Werkstoff: NdFeB, Beschichtung Nickel (Ni-Cu-Ni)) in eine Kammer, bspw. Lysekammer eingebracht.

100 µL Enterococcus faecalis in Amies-Transportmedium (von der Firma Copan) wurden in die Lysekammer pipettiert und der mikrofluidische Träger wurde bei einer Rotationsgeschwindigkeit von 20 Hz 5 min prozessiert.

Das Lysat, das durch die Lyse entsprechend der vorliegenden Offenbarung erhalten wurde, und ein thermisches Referenzlysat, welches durch Aufheizen der E. faecalis in Amies aus derselben Probe auf 95°C für 5 min prozessiert wurde, wurden anschließend mittels qPCR analysiert. Die mechanische Lyse auf dem mikrofluidischen Träger, bspw. Disk entsprechend der vorliegenden Offenbarung zeigt dabei eine vielfach höhere Lyseeffizienz als die thermische Referenzlyse.

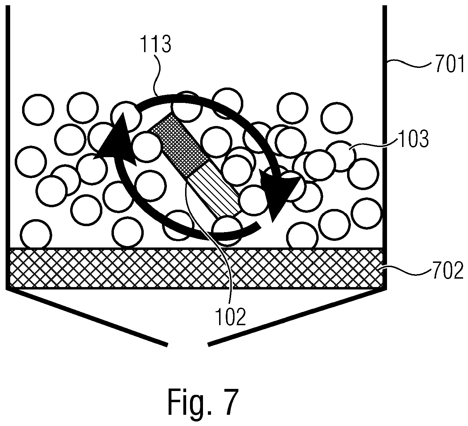

Fig. 7 zeigt eine schematische Seitenansicht eines Beispiels einer Kammer 701 einer Lysevorrichtung, die einen magnetischen Aktuator 102, Lysepartikel 103, sowie eine Membran 702 beinhaltet. Die Rotation des magnetischen Aktuators 102 im Betrieb ist durch Pfeile 113 angedeutet. Die Membran 702 kann ausgelegt sein, um Mikroorganismen aus einem größeren Volumen vor der Lyse anzureichern und anschließend die Mikroorganismen direkt auf der Membran 702 zu lysieren. Die Membran 702 kann dabei bspw. eine Filtermembran, oder z.B. ein Sterilfilter sein. Zum Beispiel können dadurch Bakterien bspw. auf der Oberfläche eines Sterilfilters angereichert werden und dadurch erreichbar für den mechanischen Eintrag der Partikel. Beispiele umfassen dabei insbesondere Lysekammern mit integriertem Sterilfilter.

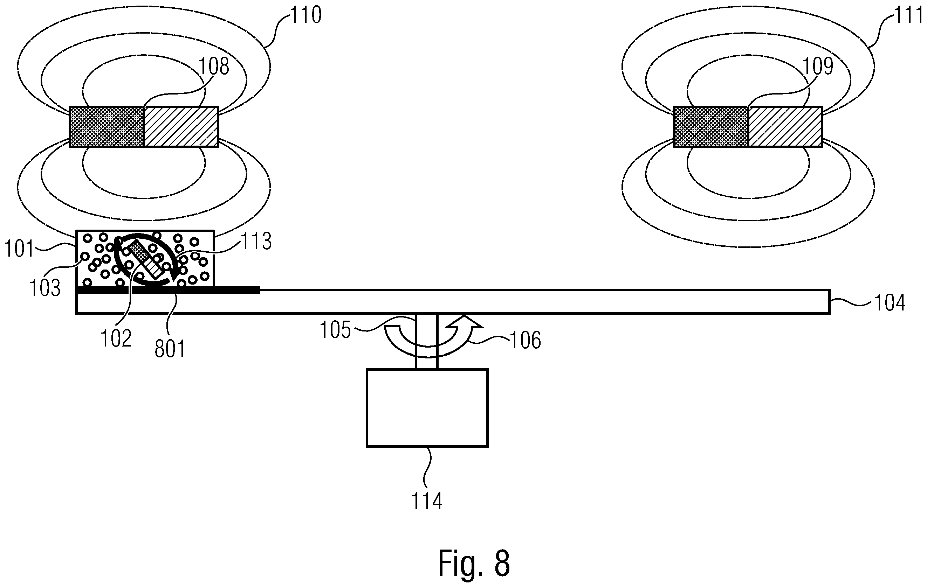

Fig. 8 zeigt eine schematische Seitenansicht eines Beispiels der Lysevorrichtung mit einer Temperiereinrichtung 801. Die Darstellung entspricht Fig. 1b, mit Ausnahme der zusätzlichen Temperiereinrichtung 801. Gleiche Elemente sind daher mit gleichen Bezugszeichen versehen. Die Temperiereinrichtung 801 befindet sich unterhalb der Kammer 102 auf dem Träger 104. Im Betrieb kann durch die Temperiereinrichtung 801 die mechanische Lyse durch einen thermischen Eintrag unterstützt werden. Die Temperiereinrichtung kann bspw. ebenfalls dazu genutzt werden, den Magneten innerhalb der Kammer über seine Curie-Temperatur zu erhitzen, sodass die Lyse beendet werden kann. Eine solche Abschaltung der Lyse ist bspw. vorteilhaft, um unabhängig von der rotatorischen Relativbewegung zwischen der Kammer 101 und den Magnetelementen 108, 109, ohne zusätzliche Betätigungsmittel eine Möglichkeit zu schaffen die Lyse zu stoppen.

Eine Umsetzungsmöglichkeit für eine Temperiereinrichtung besteht in einer Kontaktheizung. Bei Beispielen entsprechend der vorliegenden Offenbarung besteht die Möglichkeit die Lysekammer so zu positionieren, dass ein Temperatureintrag durch eine Kontaktheizung realisiert werden kann. Der Temperatureintrag könnte bspw. zwischen Umgebungstemperatur bis 120°C eingestellt werden. Dadurch kann die mechanische Lyse zusätzlich durch einen thermischen Eintrag unterstützt werden.

Bei Beispielen kann es sich bei der Temperiereinrichtung auch um eine Kontaktheizung unter der Lysekammer durch eine Heizzone handeln.

Beispiele der vorliegenden Offenbarung umfassen zwei externe statische Magnete (z.B. Neodym N45) welche über eine Halterung oberhalb oder unterhalb einer Disk (Scheibe) positioniert sein können (bspw. entsprechend Fig 1). Die Polung der externen Magnete sind dabei bspw. in Bezug auf eine rotierende Lysekammer entgegengesetzt zueinander angeordnet. Diese bestimmte Polung kann auf unterschiedliche Weise realisiert werden (bspw. entsprechend Fig. 2) und ist bei Beispielen entscheidend für die Rotationsbewegung um die eigene Achse des magnetischen Aktuators. Während einer Lysephase kann der Abstand zwischen dem Magneten und der Lysekammer in z-Richtung, bspw. vertikal zur Drehebene bspw. zwischen 0,1 mm - 50 mm betragen. Die externen Magnete können ferner auf Radien, die maximal einen Abstand von 30 mm zum Radius der Lysekammer aufweisen, positioniert sein. Innerhalb der Lysephase kann die Disk bspw. bei 0,5 Hz - 40 Hz rotieren. Der magnetische Aktuator kann z.B. als Neodym-Stabmagnet bspw. mit einer Magnetisierung N48, einer Länge von 2 mm bis 4 mm und einem Durchmesser von 2 mm bis 4 mm realisiert werden. Des Weiteren können Lysepartikel durch Glaspartikel mit einem Durchmesser von bspw. 0,15 mm bis 0,5 mm oder 0,15 mm bis 0,2 mm gebildet sein.

Weitere Beispiele gemäß der vorliegenden Offenbarung umfassen Lysevorrichtungen zur mechanischen Lyse in einer zentrifugal-mikrofluidischen Kartusche mit einer Lysekammer, enthaltend Lysepartikel und einen Permanentmagneten, mindestens zwei externe Magnete, wobei relativ zum Magnet in der Kammer, bspw. zum Magnet in der Disk, bzw. zur Lysekammer, die externen Magnete entgegengesetzt gepolt sind, um eine Rotation des Magneten innerhalb der Lysekammer zu bewirken, wobei die Größe der Lysekammer in zwei Raumrichtungen (Δr, Δϕ), bspw. radial und azimutal bzgl. der Drehebene oder (Δz, Δϕ), bspw. vertikal und azimutal bzgl. der Drehebene oder (Δz, Δr), bspw. vertikal und radial bzgl. der Drehebene mindestens die Größe der Länge des Magneten hat (Drehung in Ebene um z-Achse oder Drehung um Radiusvektor des rotierenden Systems r).

Weitere Beispiele umfassen Lysevorrichtungen, bei denen sich die externen Magnetelemente über der Kammer bzw. der Disk in einem Abstand in z-Richtung, bspw. vertikal zur Rotationsebene von maximal 5 cm befinden und nicht mehr als 5 cm von dem Radius des internen magnetischen Aktuators abweichen.

Weitere Beispiele umfassen Lysevorrichtungen, bei denen die Größe der Lysekammer in drei orthogonale Raumrichtungen (x,y,z) mindestens etwa die Länge der längsten Diagonale des magnetischen Aktuators hat (Freie Drehung; Etwas kleiner als der Magnet in z-Richtung, bspw. vertikal zur Rotationsebenen bis zu bspw. - 20% ist dabei ebenfalls möglich).

Weitere Beispiele umfassen Lysevorrichtungen, bei denen die Lyse bei einer (kontinuierlichen) Drehfrequenz von mindestens 2 Hz stattfindet. Weitere Beispiele umfassen Lysevorrichtungen, bei denen die Lyse bei einer (kontinuierlichen) Drehfrequenz von maximal 30 Hz stattfindet. Weitere Beispiele umfassen Lysevorrichtungen, bei denen die Kammer Lysepartikel in einer Größe von < 0,5 mm enthält. Weitere Beispiele umfassen Lysevorrichtungen, bei denen ein Abschalten der Lyse durch Wegbewegen der externen Magnetelemente möglich ist. Weitere Beispiele umfassen Lysevorrichtungen, bei denen die externen Magnetelemente in einen Winkel von 20-180° angeordnet sind. Weitere Beispiele umfassen Lysevorrichtungen, bei denen in die Lysekammer ein Temperatureintrag umgesetzt werden kann, um die Lysefunktion thermisch zu unterstützen.

### Vorteile der vorliegenden Offenbarung

Die induzierte translatorische- und rotatorische Bewegung des Magneten innerhalb der Kammer, bspw. eines magnetischen Aktuators durch unterschiedliche Polung der externen, bspw. statischen Magnetelemente in Bezug auf die Kammer, bspw. Lysekammer führt zu einer wesentlich besseren Durchmischung der Probe in der Kammer, insbesondere auch mit Partikeln in der Kammer. So kann im statistischen Mittel ein größerer Teil der Mikroorganismen Reibe-, Stoß- und Schwerbewegungen ausgesetzt werden. Dadurch kann eine sehr effiziente Lyse realisiert werden. In molekular diagnostischen (Schnell-) Tests ist der Faktor "Time-to-result" (Zeit-bis-Ergebnis), welcher die benötigte Zeit der Proben-Vorbereitung und -Analyse umfasst, ein entscheidender Faktor. Durch eine effiziente und schnelle Lyse lässt sich in diesem essentiellen Schritt sehr viel Zeit einsparen.

Durch die beschriebene Anordnung von externen Magnetelementen und der Kammer, bspw. Lysekammer, benötigt eine Lysevorrichtung entsprechend der vorliegenden Offenbarung in entsprechender Konfiguration sehr wenig Platz in radialer und azimutaler Richtung. Da der Platz in radialer und azimutaler Richtung auf einem mikrofluidischen Testträger sehr wertvoll ist, um eine Gesamtintegration von Probenvorbereitung und -analyse zu bewerkstelligen, ist dies ein weiterer Vorteil im Vergleich zum Stand der Technik.

Obwohl einige Aspekte der vorliegenden Offenbarung als Merkmale im Zusammenhang einer Vorrichtung beschrieben wurden, ist es klar, dass eine solche Beschreibung ebenfalls als eine Beschreibung entsprechender Verfahrensmerkmale betrachtet werden kann. Obwohl einige Aspekte als Merkmale im Zusammenhang mit einem Verfahren beschrieben wurden, ist klar, dass eine solche Beschreibung auch als eine Beschreibung entsprechender Merkmale einer Vorrichtung bzw. der Funktionalität einer Vorrichtung betrachtet werden können.

In der vorhergehenden detaillierten Beschreibung wurden teilweise verschiedene Merkmale in Beispielen zusammen gruppiert, um die Offenbarung zu rationalisieren. Diese Art der Offenbarung soll nicht als die Absicht interpretiert werden, dass die beanspruchten Beispiele mehr Merkmale aufweisen als ausdrücklich in jedem Anspruch angegeben sind. Vielmehr kann, wie die folgenden Ansprüche wiedergeben, der Gegenstand in weniger als allen Merkmalen eines einzelnen offenbarten Beispiels liegen. Folglich werden die folgenden Ansprüche hiermit in die detaillierte Beschreibung aufgenommen, wobei jeder Anspruch als ein eigenes separates Beispiel stehen kann. Während jeder Anspruch als ein eigenes separates Beispiel stehen kann, sei angemerkt, dass, obwohl sich abhängige Ansprüche in den Ansprüchen auf eine spezifische Kombination mit einem oder mehreren anderen Ansprüchen zurückbeziehen, andere Beispiele auch eine Kombination von abhängigen Ansprüchen mit dem Gegenstand jedes anderen abhängigen Anspruchs oder einer Kombination jedes Merkmals mit anderen abhängigen oder unabhängigen Ansprüchen umfassen. Solche Kombinationen seien umfasst, es sei denn es ist ausgeführt, dass eine spezifische Kombination nicht beabsichtigt ist. Ferner ist beabsichtigt, dass auch eine Kombination von Merkmalen eines Anspruchs mit jedem anderen unabhängigen Anspruch umfasst ist, selbst wenn dieser Anspruch nicht direkt abhängig von dem unabhängigen Anspruch ist.

Die oben beschriebenen Beispiele sind nur darstellend für die Grundsätze der vorliegenden Offenbarung. Es ist zu verstehen, dass Modifikationen und Variationen der Anordnungen und der Einzelheiten, die beschrieben sind, für Fachleute offensichtlich sind. Es ist daher beabsichtigt, dass die Offenbarung nur durch die beigefügten Patentansprüche und nicht durch die spezifischen Einzelheiten, die zum Zwecke der Beschreibung und Erklärung der Beispiele dargelegt sind, begrenzt ist.

### Referenzen

[1] Kido, Horacio, et al. "A novel, compact disk-like centrifugal microfluidics system for cell lysis and sample homogenization." Colloids and Surfaces B: Biointerfaces 58.1 (2007): 44-51.
[2] Siegrist, Jonathan, et al. "Validation of a centrifugal microfluidic sample lysis and homogenization platform for nucleic acid extraction with clinical samples." Lab on a Chip 10.3 (2010): 363-371.

## Patentansprüche

1. Lysevorrichtung (100) mit den folgenden Merkmalen:
einer Kammer (101) zur Aufnahme einer Probe;
zumindest einem magnetischen Aktuator (102), der sich innerhalb der Kammer befindet;
zumindest zwei außerhalb der Kammer angeordneten Magnetelementen (108, 109), und
einer Antriebseinrichtung (114) zum Bewirken einer rotatorischen Relativbewegung zwischen der Kammer und den außerhalb der Kammer angeordneten Magnetelementen, durch die die Kammer nacheinander die außerhalb der Kammer angeordneten Magnetelemente passiert, wobei die Polung der Magnetelemente in Bezug auf die Kreisbahn der rotatorischen Relativbewegung entgegengesetzt ist, so dass der in der Kammer angeordnete magnetische Aktuator sowohl translatorisch als auch rotatorisch, um eine eigene Achse des magnetischen Aktuators, bewegt wird, um eine Lyse der Probe zu bewirken,
wobei die Kammer ausgelegt ist, um zu ermöglichen, dass sich der zumindest eine magnetische Aktuator, der sich innerhalb der Kammer befindet, sowohl translatorisch, als auch rotatorisch, um eine eigene Achse des magnetischen Aktuators, bewegt.

2. Lysevorrichtung nach Anspruch 1, wobei die Magnetelemente magnetische Pole sind oder wobei jedes Magnetelement ein Magnet ist.

3. Lysevorrichtung nach einem der vorigen Ansprüche, die ausgelegt ist, um das magnetische Feld, das von den zumindest zwei außerhalb der Kammer angeordneten Magnetelementen auf den in der Kammer angeordneten magnetischen Aktuator wirkt, unabhängig von der rotatorischen Relativbewegung zumindest zu reduzieren; und
die ferner Betätigungsmittel (501) aufweist, um den Abstand zwischen der Kammer und
den zumindest zwei außerhalb der Kammer angeordneten Magnetelementen, unabhängig von der rotatorischen Relativbewegung, zu verändern.

4. Lysevorrichtung nach Anspruch 3, wobei die Betätigungsmittel ausgelegt sind, um die zumindest zwei außerhalb der Kammer angeordneten Magnetelemente senkrecht zur Drehebene zu bewegen.

5. Lysevorrichtung nach Anspruch 3 oder 4, wobei die Betätigungsmittel ausgelegt sind, um die zumindest zwei außerhalb der Kammer angeordneten Magnetelemente parallel zur Drehebene, unabhängig von der rotatorischen Relativbewegung zu bewegen.

6. Lysevorrichtung nach einem der vorigen Ansprüche, bei der die zumindest zwei außerhalb der Kammer angeordneten Magnetelemente steuer- und/oder regelbare Elektromagnete sind.

7. Lysevorrichtung nach Anspruch 6, die ausgelegt ist, um das magnetische Feld, das von den zumindest zwei außerhalb der Kammer angeordneten Magnetelementen auf den in der Kammer angeordneten magnetischen Aktuator wirkt, unabhängig von der rotatorischen Relativbewegung zumindest zu reduzieren.

8. Lysevorrichtung nach einem der vorigen Ansprüche, wobei die Lysevorrichtung zumindest einen Lysepartikel (103), der sich in der Kammer befindet, aufweist.

9. Lysevorrichtung nach Anspruch 8, wobei der zumindest eine Lysepartikel maximale Abmessungen von weniger als 0,5 mm aufweist.

10. Lysevorrichtung nach einem der vorigen Ansprüche, wobei die zumindest zwei außerhalb der Kammer angeordneten Magnetelemente dazu ausgebildet sind, um zum Zeitpunkt der Lyse stationär zu sein und die Antriebseinrichtung dazu ausgelegt ist, die Kammer bezüglich einer Drehachse, relativ zu den außerhalb der Kammer angeordneten Magnetelementen, zu rotieren.

11. Lysevorrichtung nach einem der vorigen Ansprüche, wobei die Kammer eine Membran (702) aufweist.

12. Lysevorrichtung nach einem der vorigen Ansprüche, wobei die Lysevorrichtung eine Temperiereinrichtung (801) aufweist, die ausgelegt ist, um die Temperatur der Kammer zu verändern.

13. Lysevorrichtung nach einem der vorigen Ansprüche, wobei die zumindest zwei Magnetelemente außerhalb der Kammer in einem Winkel in der Rotationsebene von 20° bis 180° zueinander angeordnet sind.

14. Lysevorrichtung nach einem der vorigen Ansprüche, wobei die Antriebseinrichtung dazu ausgebildet ist, um die rotatorische Relativbewegung mit einer Drehfrequenz von 0,5 Hz bis 40 Hz, vorzugsweise von 2 Hz bis 30 Hz bereitzustellen.

15. Lyseverfahren mit den folgenden Schritten:
Einbringen einer Probe in eine Kammer, wobei sich in der Kammer zumindest ein magnetischer Aktuator befindet und wobei die Kammer ausgelegt ist, um zu ermöglichen, dass sich der magnetische Aktuator, der sich innerhalb der Kammer befindet, sowohl translatorisch, als auch rotatorisch, um eine eigene Achse des magnetischen Aktuators, bewegt;
Bewirken einer rotatorischen Relativbewegung zwischen der Kammer, in der sich die Probe und zumindest ein magnetischer Aktuator befindet, und zwischen zumindest zwei außerhalb der Kammer angeordneten Magnetelementen, wobei die Polung der zumindest zwei Magnetelemente außerhalb der Kammer in Bezug auf die Kreisbahn der rotatorischen Relativbewegung entgegengesetzt ist, so dass der in der Kammer angeordnete magnetische Aktuator sowohl translatorisch als auch rotatorisch, um eine eigene Achse des magnetischen Aktuators, bewegt wird, um eine Lyse der Probe zu bewirken.

## Claims

1. Lysis apparatus (100), comprising:
a chamber (101) for receiving a sample;
at least one magnetic actuator (102) located within the chamber;
at least two magnetic elements (108, 109) arranged outside the chamber and
driving means (114) for effecting a rotational relative movement between the chamber and the magnetic elements arranged outside the chamber, by which the chamber successively passes the magnetic elements located outside the chamber, wherein the polarity of the magnetic elements is opposite with respect to the circular path of the rotational relative movement, such that the magnetic actuator arranged within the chamber is moved both translationally and rotationally around an own axis of the magnetic actuator to effect lysis of the sample,
wherein the chamber is configured to enable the at least one magnetic actuator located within the chamber to move both translationally and rotationally around an own axis of the magnetic actuator.

2. Lysis apparatus according to claim 1, wherein the magnetic elements are magnetic poles or wherein each magnetic element is a magnet.

3. Lysis apparatus according to one of the preceding claims, which is configured to at least reduce the magnetic field acting on the magnetic actuator arranged within the chamber from the at least two magnetic elements arranged outside the chamber, independent of the rotational relative movement, and
further comprising actuating means (501) to change the distance between the chamber and the at least two magnetic elements arranged outside the chamber, independent of the rotational relative movement.

4. Lysis apparatus according to claim 3, wherein the actuating means is configured to move the at least two magnetic elements arranged outside the chamber perpendicular to the plane of rotation.

5. Lysis apparatus according to claim 3 or 4, wherein the actuating means is configured to move the at least two magnetic elements arranged outside the chamber parallel to the plane of rotation, independent of the rotational relative movement

6. Lysis apparatus according to one of the preceding claims, wherein the at least two magnetic elements arranged outside the chamber are controllable and/or variable electromagnets.

7. Lysis apparatus according to claim 6, which is configured to at least reduce the magnetic field acting on the magnetic actuator arranged within the chamber from the at least two magnetic elements arranged outside the chamber, independent of the relative rotational motion.

8. Lysis apparatus according to one of the preceding claims, wherein the lysis apparatus comprises at least one lysis particle (103) located within the chamber.

9. Lysis apparatus according to claim 8, wherein the at least one lysis particle comprises maximum dimensions of less than 0.5 mm.

10. Lysis apparatus according to one of the preceding claims, wherein the at least two magnetic elements arranged outside the chamber are configured to be stationary at the time of the lysis and the driving means is configured to rotate the chamber with respect to an axis of rotation relative to the magnetic elements located outside the chamber.

11. Lysis apparatus according to one of the preceding claims, wherein the chamber comprises a diaphragm (702).

12. Lysis apparatus according to one of the preceding claims, wherein the lysis apparatus comprises tempering means (801) configured to change the temperature of the chamber.

13. Lysis apparatus according to one of the preceding claims, wherein the at least two magnetic elements outside the chamber are arranged at an angle in the plane of rotation of 20° to 180° to each other.

14. Lysis apparatus according to one of the preceding claims, wherein the driving means is configured to provide the rotational relative movement with a rotational frequency of 0.5 Hz to 40 Hz, preferably of 2 Hz to 30 Hz,

15. Lysis method, comprising:
introducing a sample into a chamber, wherein at least one magnetic actuator is located in the chamber and wherein the chamber is configured to enable the magnetic actuator located within the chamber to move both translationally and rotationally around an own axis of the magnetic actuator;
effecting a rotational relative movement between the chamber wherein the sample and at least one magnetic actuator are located, and between at least two magnetic elements located outside the chamber, wherein the polarity of the at least two magnetic elements outside the chamber is opposite with respect to the circular path of the rotational relative movement, such that the magnetic actuator located within the chamber is moved both translationally and rotationally around an own axis of the magnetic actuator to effect lysis of the sample.

## Revendications

1. Dispositif de lyse (100) aux caractéristiques suivantes:
une chambre (101) destinée à recevoir un échantillon;
au moins un actionneur magnétique (102) qui est situé à l'intérieur de la chambre;
au moins deux éléments magnétiques (108, 109) disposés à l'extérieur de la chambre, et
un moyen d'entraînement (114) destiné à provoquer un mouvement relatif de rotation entre la chambre et les éléments magnétiques disposés à l'extérieur de la chambre, au moyen duquel la chambre passe devant, l'un après l'autre, des éléments magnétiques disposés à l'extérieur de la chambre, où la polarité des éléments magnétiques est opposée en ce qui concerne la trajectoire circulaire du mouvement relatif de rotation, de sorte que l'actionneur magnétique disposé dans la chambre soit déplacé tant en translation qu'en rotation autour d'un axe propre de l'actionneur magnétique, pour provoquer une lyse de l'échantillon,
dans lequel la chambre est conçue pour permettre que l'au moins un actionneur magnétique que se situe à l'intérieur de la chambre se déplace tant en translation qu'en rotation autour d'un axe propre de l'actionneur magnétique.

2. Dispositif de lyse selon la revendication 1, dans lequel les éléments magnétiques sont des pôles magnétiques ou dans lequel chaque élément magnétique est un aimant.

3. Dispositif de lyse selon l'une des revendications précédentes, qui est conçu pour au moins réduire le champ magnétique qui agit des au moins deux éléments magnétiques disposés à l'extérieur de la chambre sur l'actionneur magnétique disposé dans la chambre, de manière indépendante du mouvement relatif de rotation; et
qui présente par ailleurs des moyens d'actionnement (501) pour modifier la distance entre la chambre et les au moins deux éléments magnétiques disposés à l'extérieur de la chambre, de manière indépendante du mouvement relatif de rotation.

4. Dispositif de lyse selon la revendication 3, dans lequel les moyens d'actionnement sont conçus pour déplacer les au moins deux éléments magnétiques disposés à l'extérieur de la chambre de manière perpendiculaire au plan de rotation.

5. Dispositif de lyse selon la revendication 3 ou 4, dans lequel les moyens d'actionnement sont conçus pour déplacer les au moins deux éléments magnétiques disposés à l'extérieur de la chambre de manière parallèle au plan de rotation, de manière indépendante du mouvement relatif de rotation.

6. Dispositif de lyse selon l'une des revendications précédentes, dans lequel les au moins deux éléments magnétiques disposés à l'extérieur de l'enceinte sont des électro-aimants pouvant être commandés et/ou réglés.

7. Dispositif de lyse selon la revendication 6, qui est conçu pour au moins réduire le champ magnétique qui agit des au moins deux éléments magnétiques disposés à l'extérieur de la chambre sur l'actionneur magnétique disposé dans la chambre, de manière indépendante du mouvement relatif de rotation.

8. Dispositif de lyse selon l'une des revendications précédentes, dans lequel le dispositif de lyse présente au moins une particule de lyse (103) qui est située dans la chambre.

9. Dispositif de lyse selon la revendication 8, dans lequel l'au moins une particule de lyse présente des dimensions maximales de moins de 0,5 mm.

10. Dispositif de lyse selon l'une des revendications précédentes, dans lequel les au moins deux éléments magnétiques disposés à l'extérieur de la chambre sont conçus de manière à être stationnaires au moment de la lyse et le moyen d'entraînement est conçu pour faire tourner la chambre, par rapport à un axe de rotation, par rapport aux éléments magnétiques disposés à l'extérieur de la chambre.

11. Dispositif de lyse selon l'une des revendications précédentes, dans lequel la chambre présente une membrane (702).

12. Dispositif de lyse selon l'une des revendications précédentes, dans lequel le dispositif de lyse présente un moyen de régulation de la température (801) qui est conçu pour modifier la température de la chambre.

13. Dispositif de lyse selon l'une des revendications précédentes, dans lequel les au moins deux éléments magnétiques sont disposés à l'extérieur de l'enceinte selon un angle dans le plan de rotation de 20° à 180° l'un par rapport à l'autre.

14. Dispositif de lyse selon l'une des revendications précédentes, dans lequel le moyen d'entraînement est conçu pour assurer le mouvement relatif de rotation à une fréquence de rotation de 0,5 Hz à 40 Hz, de préférence de 2 Hz à 30 Hz.

15. Procédé de lyse aux étapes suivantes consistant à:
introduire un échantillon dans une chambre, où au moins un actionneur magnétique est situé dans la chambre et où la chambre est conçue pour permettre que l'actionneur magnétique qui se situe à l'intérieur de la chambre se déplace tant en translation qu'en rotation par rapport à un axe propre de l'actionneur magnétique;
provoquer un mouvement relatif de rotation entre la chambre dans laquelle se trouve l'échantillon et au moins un actionneur magnétique, et entre au moins deux éléments magnétiques disposés à l'extérieur de la chambre, où la polarité des au moins deux éléments magnétiques à l'extérieur de la chambre est opposée en ce qui concerne la trajectoire circulaire du mouvement relatif de rotation, de sorte que l'actionneur magnétique disposé dans la chambre soit déplacé tant en translation qu'en rotation autour d'un axe propre de l'actionneur magnétique, pour provoquer une lyse de l'échantillon.
